# EUROPEAN PATENT SPECIFICATION

(11) **EP 1 123 935 B1**
(45) Date of publication and mention of the grant of the patent: **13.04.2005**
(21) Application number: 01103370.1
(22) Date of filing: 14.08.1998
(51) Int. Cl.: C07D 487/04, A61K 31/495

(54) **3'-Fluorinated guanosine derivatives for the treatment or prophylaxis of HBV or retroviral infections**
3'-Fluorierte Guanosinderivate zur Behandlung oder Prophylaxe von HBV- oder retroviralen Infektionen
Dérivés de guanosine 3'-fluorés pour le traitement ou la prophylaxie des infections HBV ou retrovirales

(30) Priority: 15.08.1997 SE 9702957; 12.11.1997 SE 9704147; 13.02.1998 SE 9800452
(43) Date of publication of application: 16.08.2001
(62) Divisional of application: 98939041.4
(73) Proprietor: MEDIVIR AB, 141 44 Huddinge (SE)
(72) Inventor: Zhou, Xiao-Xiong, 141 41 Huddinge (SE); Johansson, Nils-Gunnar, 150 23 Enhörna (SE); Wähling, Horst, 127 61 Skärholmen (SE)
(74) Representative: Dehmel, Albrecht, Dr.

(56) References cited:
- EP-A- 0 694 547
- WO-A-88/00050
- WO-A-97/27194
- US-A- 4 957 924

## Description

This invention relates to the field of nucleoside analogues, such as antivirals including inhibitors of retroviral reverse transcriptase and the DNA polymerase of Hepatitis B Virus (HBV). The invention provides novel compounds with favourable pharmaceutical parameters, pharmaceutical compositions comprising these compounds and methods employing them for the preparation of a medicament for the inhibition of viral and neoplastic diseases including HBV and HIV.

### Background to the invention

International patent application WO 88/00050 describes the antiretroviral and anti-HBV activity of a series of 3'-fluorinated nucleosides, including the compounds 2',3'-dideoxy, 3'-fluoroguanosine (FLG) and 3'-fluorothymidine (FLT). The latter compound underwent clinical evaluation as an anti-HIV agent and although its antiviral activity and pharmacokinetics were good, it showed unexpected toxicity (Flexner et al, J Inf Dis 170(6) 1394-403 (1994)). The former compound FLG is very active in vitro, however, the present inventors have detected that its bioavailability is so poor - around 4% - that the in vivo utility of the compound has thus far been limited to intraperitoneally or subcutaneously administered animal models.

US patent 4,963,662 discloses generically a series of 3'-fluorinated nucleosides and corresponding triphosphates and specifically describes the preparation of the 5'-O-palmitoyl derivative of FLT, without reporting any improvement in bioavailability. International patent application WO 93 13778 describes FLG derivatives modified at the 6-position of the base, in particular with n-propoxy, cyclobutoxy, cyclopropanylamino, piperidino or pyrrolidino. International patent application no. 93 14103 describes FLG derivatives where the oxygen at the guanine 6-position is replaced with amino, ether, halo or sulphonate.

EP-A 0 694 547 describes the L-monovaline ester derived from 2-(2-amino-1,6-dihydro-6-oxo-purin-9-yl)methoxy-1,3-propanediol and its pharmaceutically acceptable salts as antiviral agents exhibiting improved absorption. A two-step method to prepare intermediates for the preparation of these esters is disclosed in WO 97/27194. US 4,957,924 relates to amino acid esters of acyclovir, pharmaceutically acceptable salts thereof, and their use to treat herpes infections.

### Brief description of the invention

In accordance with one aspect of the invention there are provided compounds of the formula I: 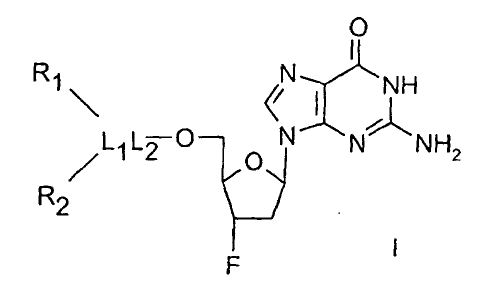 wherein:
R₁ is hydroxy, optionally having esterified thereon an aliphatic L-amino acid;
R₂ is the residue of an aliphatic L-amino acid esterified to an hydroxy function on L₁;
L₁ is a trifunctional linker group, wherein one function is R₁ and a second function is an hydroxy group to which R₂ is esterified;
L₂ is absent or a difunctional linker group;
wherein the third function on L₁; or, if present, a carboxy function on L₂; is a carboxy group esterified to the adjacent 5'-O function;
and pharmaceutically acceptable salts thereof
The invention further provides pharmaceutical compositions comprising the compounds and salts of formula I and pharmaceutically acceptable carriers or diluents therefor. The invention also extends to the use of the compounds or salts of formula I in the preparation of a medicament for the treatment of retroviral or HBV infections.

In treating conditions caused by retroviruses such as HIV, or HBV, the compounds or salts of formula I are preferably administered in an amount of 50 to 1 500 mg once, twice or three times per day, especially 100 to 700 mg twice or thrice daily. It is desirable to achieve serum levels of the active metabolite of 0.01 to 100 µg/ml, especially 0.1 to 5 µg/ml.

Suitable aliphatic amino acids for R₂ and, if present R₁, include L-alanine, L-leucine, L-isoleucine and most preferably L-valine. For ease of synthesis it is preferred that both R₂ and R₁ are residues of aliphatic amino acids, preferably the same residue.

The expression trifunctional in the context of the first linker group L₁ means that the linker has at least three functional groups, including at least two functional groups derived from respective hydroxy groups, the hydroxy function(s) being available for esterification with the carboxy functions of R₁ and R₂. Where R₁ itself defines an hydroxy, one of said functions on the trifunctional linker simply comprises this hydroxy, amine or carboxy group.

The trifunctional linker further comprises a third functional group, a carboxy group, for linkage with either the optional second linker group L₂ illustrated in more detail below, or the hydroxy group at the 5' position of the mother nucleoside, 2',3'-dideoxy-3'-fluoroguanosine. If L₂ is absent, this third functional group on first linker L₁ will typically comprise a carboxyl function which can esterify with the 5'-O group of the nucleoside analogue.

Useful trifunctional L₁ group, especially for esterifying directly to the nucleoside include linkers of the formula IIa or IIb: 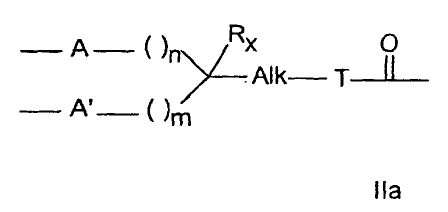 where A and A' define a respective ester linkage between an hydroxy on the linker and the carboxy on R₁ or R₂ or one of A and A' is as defined and the other is hydroxy, amino or carboxy in the event that R₁ itself is a free hydroxy group.
Rx is H or C₁-C₃ alkyl,
T is a bond, -O- or -NH-;
Alk is absent, C₁-C₄ alkyl or C₂-C₄ alkenyl, optionally substituted as described above; and
m and n are independently 0, 1 or 2.

In a preferred embodiment of this aspect of the invention, the R₁ or R₂ groups are each esterified to a respective one of the leftmost functional hydroxy groups (viz A and A') of Formula IIa, while the carbonyl moiety to the right is esterified, optionally via a second linker group L₂, to the 5'-O-group of the nucleoside.

Alternatively the L₁ group may comprise a linker of the formula IIb: 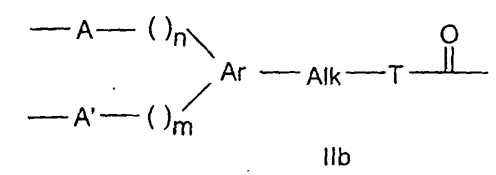 where Ar is a saturated or unsaturated, preferably monocyclic carbo- or heterocycle with 5 or 6 ring atoms; and
A, A', T, Alk, m and n are as defined above.

In Formula IIb, Ar is preferably an aromatic group such as pyridine or especially phenyl, such as aromatic moieties wherein the arms bearing the R₁ and R₂ groups are respectively para and ortho, meta and ortho, both ortho, or preferably para and meta, both para or both meta to the remainder of the linker.

In formulae IIa and IIb, the following combinations of m, n and Alk are presently favoured:

| m | n | Alk |
|---|---|---|
| 1 | 0 | methylene |
| 1 | 0 | ethylene |
| 1 | 1 | absent |
| 1 | 1 | methylene |
| 1 | 1 | ethylene |
| 1 | 1 | propylene |
| 1 | 2 | absent |
| 1 | 2 | methylene |
| 1 | 1 | ethenylene |
| 1 | 1 | propenylene |

As R₁ and R₂ may have different structures, it will be apparent that many L₁ groups, particularly those of formula IIa, will define chiral structures and the invention includes all enantiomers thereof, as racemates or as preparations of > 80%, preferably > 95% enantiomerically pure compound.

A particularly preferred group of trifunctional linkers comprise glycerol derivatives of the formula IIc 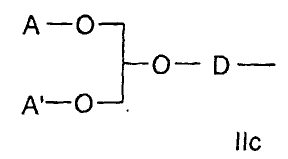 where A is hydrogen, the acyl residue of an aliphatic L-amino acid ester, A' is the acyl residue of an aliphatic amino acid residue and D is a C₂-C₆ saturated or unsaturated dicarboxylic acid residue. Trifunctional linkers of the formula IIc are hydrolysed or otherwise break down in vivo to release the nature identical compounds glycerol, the L-amino acid, the fatty acid (if present) and the dicarboxylic acid, each of which are generally safely metabolised and/or excreted by the body. Preferably A and A' are both residues of an aliphatic amino acid, most preferably the same residue, particularly residues of L-valine or L-isoleucine.

In the event that the dicarboxylic acid moiety in the derivative of formula IIc is esterified directly to the 5' hydroxy function (or equivalent) on the nucleoside, an alternative analysis would be to define the glycerol moiety as trifunctional linker L₁ and the dicarboxylic acid moiety as difunctional linker L₂.

Particularly preferred dicarboxylic acid residues include those derived from oxalic, malonic, tartronic, succinic, maleic, fumaric, malic, tartaric, glutaric, glutaconic, citraconic, itaconic, ethidine-malonic, mesaconic, adipic, allylmalonic, propylidenemalonic, hydromuconic, pyrocinchonic and muconic acids and the like. The dicarboxylic acid residue may be optionally substituted, for example with the substituents listed above in respect of R₁ as a fatty acid. Hydroxy substituents can in turn be esterified with a further L-amino acid or fatty acid residue.

Several of the above mentioned dicarboxylic acids can themselves define a trifunctional linker. For instance hydroxy-substituted dicarboxylic acids such as tartaric acid or malic acid offer a number of configurations within the scope of the invention. Taking tartaric acid as an example a carboxyl function is available for esterification with the 5'-hydroxyl function of a nucleoside (optionally via difunctional linker L₂). The hydroxy functions are available for esterification with the respective carboxyl functions of R₂ and an R₁ fatty acid or amino acid while the remaining carboxy group can be free, or optionally protected, for instance with a conventional pharmaceutically acceptable ester such as the methyl or ethyl ester. Alternatively the optional protection of the free carboxy function can itself comprise an ester with an R₁ fatty alcohol, with one or both hydroxyl functions being esterified to R₂:

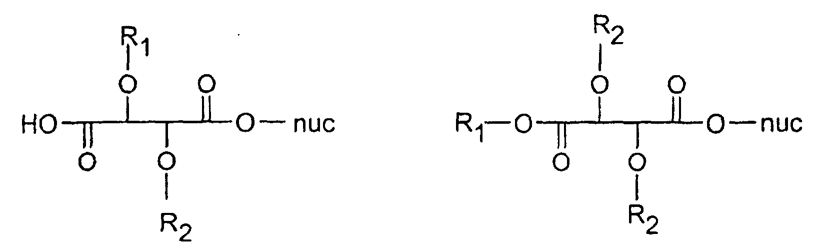

Favoured linkers of the tartaric acid series above can be generically depicted as Formula IIe: 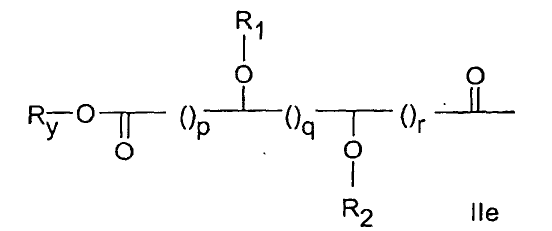 and isomers where R₁ and R₂ are reversed, where R₁ and R₂ are as shown above, p, q and r are each independently 0 to 5, preferably 0 or 1 and Ry is the free acid, an R₁ ester or a conventional pharmaceutically acceptable carboxy protecting group, such as the methyl, benzyl or especially the ethyl ester.

Favoured linkers of the malic series have the formula IIf: 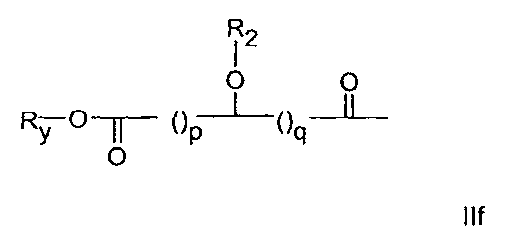 where Ry, p, q and R₂ are as defined above, preferably those where p and q are zero.

Preferred compounds of this aspect of the invention thus include:
5'-O-[3-methoxycarbonyl-2-valyloxy-propionyl]-2',3'-dideoxy-3'-fluoroguanosine,
5'-O-[3-benzyloxycarbonyl-2-valyloxy-propionyl]-2',3'-dideoxy-3'-fluoroguanosine,
5'-O-[3-methoxycarbonyl-2-isoleucoxy-propionyl]-2',3'-dideoxy-3'-fluoroguanosine,
5'-O-[3-benzyloxycarbonyl-2-isoleucyloxy-propionyl]-2',3'-dideoxy-3'-fluoroguanosine,
5'-O-[4-methoxycarbonyl-2,3-bis-valyloxy-butyryl]-2',3'-dideoxy-3'-fluoroguanosine,
5'-O-[4-benzyloxycarbonyl-2,3-bis-valyloxy-butyryl]-2',3'-dideoxy-3'-fluoroguanosine,
S'-O-[4-methoxycarbonyl-2,3-bis-isoleucyloxy-butyryl]-2',3'-dideoxy-3'-fluoroguanosine,
5'-O-[4-benzyloxycarbonyl-2,3-bis-isoleucyloxy-butyryl]-2',3'-dideoxy-3'-fluoroguanosine;
particularly those derived from L-malic acid and L-tartaric acid; and corresponding derivatives employing conventional pharmaceutically acceptable esters on the terminal carboxy function.

Particularly favoured compounds include:
5'-O-[3-ethoxycarbonyl-2-valyloxy-proplonyl]-2',3'-didepxy-3'-fluoroguanosine,
5'-O-[3-ethoxycarbonyl-2-isoleucyloxy-propionyl]-2',3'-dideoxy-3'-fluoroguanosine,
5'-O-[4-ethoxycarbonyl-2,3-bis-valyloxy-butyryl]-2',3'-dideoxy-3'-fluoroguanosine,
5'-O-[4-ethoxycarbonyl-2,3-bis-isoleucyloxy-butyryl]-2',3'-dideoxy-3'-fluoroguanosine,
especially the isomers derived from L-malic and L-tartaric acid.

As described above, a preferred group of bifunctional linkers comprises α,ω-dicarboxylic C₂-C₆ alkyl derivatives, such as succinic acid, which are optionally substituted (for instance with the substituents defined above for R₁ as a fatty acid) and/or optionally mono or polyunsaturated, such as n-3 or n-6 monounsaturated. Preferred moieties within this class are listed above.

Although the disclosure above has concentrated on glycerol L₁ groups in conjunction with dicarboxylic L₂ groups, it will be appreciated that a wide variety of trifunctional linkers are appropriate with dicarboxylic L₂ groups, for instance structures of the formula IIa and IIb above lacking the rightmost carbonyl.

The compounds of the invention can form salts which form an additional aspect of the invention. Appropriate pharmaceutically acceptable salts of the compounds of Formula I include salts of organic acids, especially carboxylic acids, including but not limited to acetate, trifluoroacetate, lactate, gluconate, citrate, tartrate, maleate, malate, pantothenate, isethionate, adipate, alginate, aspartate, benzoate, butyrate, digluconate, cyclopentanate, glucoheptanate, glycerophosphate, oxalate, heptanoate, hexanoate, fumarate, nicotinate, palmoate, pectinate, 3-phenylpropionate, picrate, pivalate, proprionate, tartrate, lactobionate, pivolate, camphorate, undecanoate and succinate, organic sulphonic acids such as methanesulphonate, ethanesulphonate, 2-hydroxyethane sulphonate, camphorsulphonate, 2-napthalenesulphonate, benzenesulphonate, p-chlorobenzenesulphonate and p-toluenesulphonate; and inorganic acids such as hydrochloride, hydrobromide, hydroiodide, sulphate, bisulphate, hemisulphate, thiocyanate, persulphate, phosphoric and sulphonic acids. The compounds of Formula I may in some cases be isolated as the hydrate.

The term "N-protecting group" or "N-protected" as used herein refers to those groups intended to protect the N-terminus of an amino acid or peptide or to protect an amino group against undesirable reactions during synthetic procedures. Commonly used N-protecting groups are disclosed in Greene, "Protective Groups in Organic Synthesis" (John Wiley & Sons, New York, 1981), which is hereby incorporated by reference. N-protecting groups include acyl groups such as formyl, acetyl, propionyl, pivaloyl, t-butylacetyl, 2-chloroacetyl, 2-bromoacetyl, trifluoroacetyl, trichloroacetyl, phthalyl, o-nitrophenoxyacetyl, α-chlorobutyryl, benzoyl, 4-chlorobenzoyl, 4-bromobenzoyl, 4-nitrobenzoyl, and the like; sulfonyl groups such as benzenesulfonyl, p-toluenesulfonyl, and the like, carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxycarbonyl, t-butoxycarbonyl, diisopropylmethoxycarbonyl, isopropyloxycarbonyl, ethoxycarbonyl, methoxycarbonyl, allyloxycarbonyl, 2,2,2-trichloroethoxycarbonyl, phenoxycarbonyl, 4-nitrophenoxycarbonyl, fluorenyl-9-methoxycarbonyl, cyclopentyl-oxycarbonyl, adamantyloxycarbonyl, cyclohexyloxycarbonyl, phenylthiocarbonyl, and the like; alkyl gropus such as benzyl, triphenylmethyl, benzyloxymethyl and the like; and silyl groups such as trimethylsilyl and the like. Favoured N-protecting, groups include formyl, acetyl, allyl, F-moc, benzoyl, pivaloyl, t-butylacetyl, phenylsulfonyl, benzyl, t-butoxycarbonyl (BOC) and benzyloxycarbonyl (Cbz).

Hydroxy and/or carboxy protecting groups are also extensively reviewed in Greene ibid and include ethers such as methyl, substituted methyl ethers such as methoxymethyl, methylthiomethyl, benzyloxymethyl, t-butoxymethyl, 2-methoxyethoxymethyl and the like, silyl ethers such as trimethylsilyl (TMS), t-butyldimethylsilyl (TBDMS) tribenzylsilyl, triphenylsilyl, t-butyldiphenylsilyl triisopropyl silyl and the like, substituted ethyl ethers such as 1-ethoxymethyl, 1-methyl-1-methoxyethyl, t-butyl, allyl, benzyl, p-methoxybenzyl, diphenylmethyl, triphenylmethyl and the like, aralkyl groups such as trityl, and pixyl (9-hydroxy-9-phenylxanthene derivatives, especially the chloride). Ester hydroxy protecting groups include esters such as formate, benzylformate, chloroacetate, methoxyacetate, phenoxyacetate, pivaloate, adamantoate, mesitoate, benzoate and the like. Carbonate hydroxy protecting groups include methyl vinyl, allyl, cinnamyl, benzyl and the like.

In keeping with the usual practice with retroviral and HBV inhibitors it is advantageous to co-administer one to three or more additional antivirals, such as AZT, ddI, ddC, d4T, 3TC, H2G, foscarnet, ritonavir, indinavir, saquinavir, nevirapine, delaviridine, Vertex VX 478 or Agouron AG1343 and the like in the case of HIV or lamivudine, interferon, famciclovir etc in the case of HBV. Such additional antivirals will normally be administered at dosages relative to each other which broadly reflect their respective therapeutic values. Molar ratios of 100:1 to 1:100, especially 25:1 to 1:25, relative to the compound or salt of formula I will often be convenient. Administration of additional antivirals is generally less common with those antiviral nucleosides intended for treating herpes infections.

While it is possible for the active agent to be administered alone, it is preferable to present it as part of a pharmaceutical formulation. Such a formulation will comprise the above defined active agent together with one or more acceptable carriers/excipients and optionally other therapeutic ingredients. The carrier(s) must be acceptable in the sense of being compatible with the other ingredients of the formulation and not deleterious to the recipient.

The formulations include those suitable for rectal, nasal, topical (including buccal and sublingual), vaginal or parenteral (including subcutaneous, intramuscular, intravenous and intradermal) administration, but preferably the formulation is an orally administered formulation. The formulations may conveniently be presented in unit dosage form, e.g. tablets and sustained release capsules, and may be prepared by any methods well known in the art of pharmacy.

Such methods include the step of bringing into association the above defined active agent with the carrier. In general, the formulations are prepared by uniformly and intimately bringing into association the active agent with liquid carriers or finely divided solid carriers or both, and then if necessary shaping the product. The invention extends to methods for preparing a pharmaceutical composition comprising bringing a compound of Formula I or its pharmaceutically acceptable salt in conjunction or association with a pharmaceutically acceptable carrier or vehicle. If the manufacture of pharmaceutical formulations involves intimate mixing of pharmaceutical excipients and the active ingredient in salt form, then it is often preferred to use excipients which are non-basic in nature, i.e. either acidic or neutral.

Formulations for oral administration in the present invention may be presented as discrete units such as capsules, cachets or tablets each containing a predetermined amount of the active agent; as a powder or granules; as a solution or a suspension of the active agent in an aqueous liquid or a non-aqueous liquid; or as an oil-in-water liquid emulsion or a water in oil liquid emulsion and as a bolus etc.

With regard to compositions for oral administration (e.g. tablets and capsules), the term suitable carrier includes vehicles such as common excipients e.g. binding agents, for example syrup, acacia, gelatin, sorbitol, tragacanth, polyvinylpyrrolidone (Povidone), methylcellulose, ethylcellulose, sodium carboxymethylcellulose, hydroxypropylmethylcellulose, sucrose and starch; fillers and carriers, for example com starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate stearic acid, silicone fluid, talc waxes, oils and colloidal silica. Flavouring agents such as peppermint, oil of wintergreen, cherry flavouring or the like can also be used. It may be desirable to add a colouring agent to make the dosage form readily identifiable. Tablets may also be coated by methods well known in the art.

A tablet may be made by compression or moulding, optionally with one or more accessory ingredients. Compressed tablets may be prepared by compressing in a suitable machine the active agent in a free flowing form such as a powder or granules, optionally mixed with a binder, lubricant, inert diluent, preservative, surface-active or dispersing agent. Moulded tablets may be made by moulding in a suitable machine a mixture of the powdered compound moistened with an inert liquid diluent. The tablets may be optionally be coated or scored and may be formulated so as to provide slow or controlled release of the active agent.

Other formulations suitable for oral administration include lozenges comprising the active agent in a flavoured base, usually sucrose and acacia or tragacanth; pastilles comprising the active agent in an inert base such as gelatin and glycerol, or sucrose and acacia; and mouthwashes comprising the active agent in a suitable liquid carrier.

A still further aspect of the invention provides a method for the preparation of a compound of Formula I or Ic comprising the acylation of the nucleoside, represented here by FLG, Formula V, typically at the 5' hydroxy group: 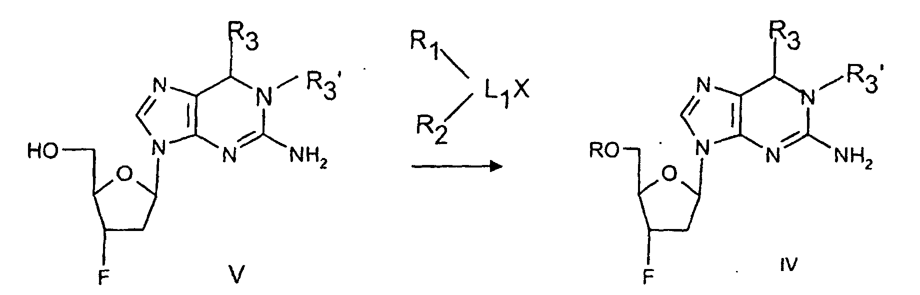 in which R₁(R₂)L₁X represents an activated acid, such as the carboxylic derivatives of Formula IIa or IIb, where R₁, R₂, and L₁ are as defined above or protected derivatives thereof. Alternatively the activated acid may comprise a compound of the formula R₁(R₂)glycerol-D-X, where R₁, R₂ and D are as defined in formula IIc or an activated Rz-O-Alk-C(=O)X derivative in the case of compounds of formula Ia. In the latter cases the linkers may be built up sequentially by first esterifying a suitably protected D or ω-hydroxy carboxylic acid to the nucleoside, deprotecting the terminal carboxy or hydroxy function and esterifying the suitably protected glycerol or Rz moiety thereon.

The activated derivative used in the acylation may comprise e.g, the acid halide, acid anhydride, activated acid ester or the acid in the presence of coupling reagent, for example dicyclohexylcarbodiimide. Representative activated acid derivatives include the acid chloride, anhydrides derived from alkoxycarbonyl halides such as isobutyloxycarbonylchloride and the like, N-hydroxysuccinamide derived esters, N-hydroxyphthalimide derived esters, N-hydroxy-5-norbomene- 2,3-dicarboxamide derived esters, 2,4,5-trichlorophenol derived esters and the like. Further activated acids include those where X in the formula RX represents an OR'moiety where R is R₂ as defined herein, and R' is, for example COCH₃, COCH₂CH₃ or COCF₃ or where X is benzotriazole.

The intermediates used in the above methods themselves define novel compounds, especially those of the formula: IIc' 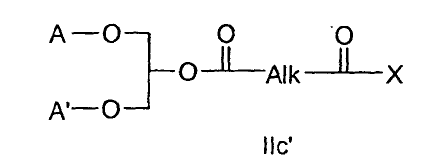 where A, A' and Alk are as defined above (A and A' being optionally protected with conventional protecting groups) and X represents the free acid or an activated acid as illustrated above.

Representative compounds of the formula IIc' include:
malonic acid 2,3-*bis*-(L-valyloxy)-propyl ester,
malonic acid 2,3-*bis*-(N-CBZ-L-valyloxy)-propyl ester,
malonic acid 2,3-*bis*-(N-Fmoc-L-valyloxy)-propyl ester,
malonic acid 2,3-*bis*-(N-Boc-L-valyloxy)-propyl ester,
malonic acid 2,3-*bis*-(L-isoleucyloxy)-propyl ester,
malonic acid 2,3-*bis*-(N-CBZ-L-isoleucyloxy)-propyl ester,
malonic acid 2,3-*bis*-(N-Fmoc-L-isoleucyloxy)-propyl ester,
malonic acid 2,3-*bis*-(N-Boc-L-isoleucyloxy)-propyl ester,
succinic acid 2,3-*bis*-(L-valyloxy)-propyl ester,
succinic acid 2,3-*bis*-(N-CBZ-L-valyloxy)-propyl ester,
succinic acid 2,3-*bis*-(N-Fmoc-L-valyloxy)-propyl ester,
succinic acid 2,3-*bis*-(N-Boc-L-valyloxy)-propyl ester,
succinic acid 2,3-*bis*-(L-isoleucyloxy)-propyl ester,
succinic acid 2,3-*bis*-(N-CBZ-L-isoleucyloxy)-propyl ester,
succinic acid 2,3-*bis*-(N-Fmoc-L-isoleucyloxy)-propyl ester,
succinic acid 2,3-*bis*-(N-Boc-L-isoleucyloxy)-propyl_{.}ester,
glutaric acid 2,3-*bis*-(L-valyloxy)-propyl ester,
glutaric acid 2,3-*bis*-(N-CBZ-L-valyIoxy)-propyl ester,
glutaric acid 2,3-*bis*-(N-Fmoc-L-valyloxy)-propyl ester,
glutaric acid 2,3-*bis*-(N-Boc-L-valyloxy)-propyl ester,
glutaric acid 2,3-*bis*-(L-isoleucyloxy)-propyl ester,
glutaric acid 2,3-*bis*-(N-CBZ-L-isoleucyloxy)-propyl ester,
glutaric acid 2,3-*bis*-(N-Fmoc-L-isoleucyloxy)-propyl ester,
glutaric acid 2,3-*bis*-(N-Boc-L-isoleucyloxy)-propyl ester,
and the corresponding acid halides, in particular the chloride, acid anhydrides and diesters of each of the above, for instance
succinic acid 2,3-*bis*-(N-CBZ-L-valyloxy)-propyl ester,4-methoxybenzyl ester
succinic acid 2,3-*bis*-(N-CBZ-L-valyloxy)-propyl ester, 1,1-dimethylethyl ester, etc.

A further preferred group of intermediates comprise those of the formula IIa': 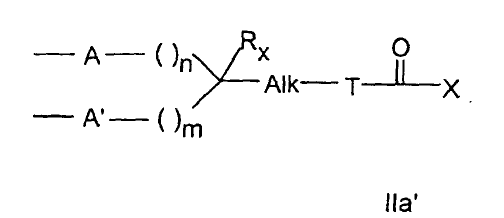 where Rₓ, Alk, m, n and T are as described above, A and A' represent acyl residues of L'-aliphatic amino acids (N-protected as necessary) esterified to hydroxy functions on the linker or one of A and A' is the acyl residue and the other is a free hydroxy group, and X represents the free acid or an activated acid as illustrated above. Preferably A and A' are the same amino acid residue.

Further novel intermediates include precursors of compounds of the formula IIe and IIf above, especially those derived from "natural" configurations such as L-malic and L-tartaric acid; for instance:
3-ethoxycarbonyl-2-valyloxy-propionic acid
3-ethoxycarbonyl-2-isoleucyloxy-propionic acid
4-ethoxycarbonyl-2,3-bis-valyloxy-butyric acid
4-ethoxycarbonyl-2,3-bis-isoleucyloxy-butyric acid
3-benzyloxycarbonyl-2-valyloxy-propionic acid
3-benzyloxycarbonyl-2-isoleucyloxy-propionic acid
4-benzyloxycarbonyl-2,3-bis-valyloxy-butyric acid
4-benzyloxycarbonyl-2,3-bis-isoleucyloxy-butyric acid, and the like;
and the corresponding activated derivatives such as the acid halides.

Preparation of 3' fluoronucleosides such as those of formula V has been extensively reviewed by Herdiwijn et al. in Nucleosides and Nucleotides 8 (1) 65-96 (1989), which is hereby incorporated by reference.

The reactive derivatives of the R₁(R₂)L₁L₂X group may be pre-formed or generated in situ by the use of reagents such as dicyclohexylcarbodiimide (DCC) or O-(1H-benzotriazol-1-yl) N,N,N',N'-tetramethyluronium tetrafluoroborate (TBTU). When an acid halide, such as the acid chloride is used, a tertiary amine catalyst, such as triethylamine, N,N'-dimethylaniline, pyridine or dimethylaminopyridine may be added to the reaction mixture to bind the liberated hydrohalic acid.

The reactions are preferably carried out in an unreactive solvent such as N,N-dimethylformamide, tetrahydrofuran, dioxane, acetonitrile or a halogenated hydrocarbon, such as dichloromethane. If desired, any of the above mentioned tertiary amine catalysts may be used as solvent, taking care that a suitable excess is present. The reaction temperature can typically be varied between 0° C and 60° C, but will preferably be kept between 5° and 50° C. After a period of 1 to 60 hours the reaction will usually be essentially complete. The progress of the reaction can be followed using thin layer chromatography (TLC) and appropriate solvent systems. In general, when the reaction is completed as determined by TLC, the product is extracted with an organic solvent and purified by chromatography and/or recrystallisation from an appropriate solvent system.

By-products where acylation has taken place on the nucleoside base can be separated by chromatography, but such misacylation can be minimized by controlled reaction conditions. These controlled conditions can be achieved, for example, by manipulating the reagent concentrations or rate of addition, especially of the acylating agent, by lowering the temperature or by the choice of solvent. The reaction can be followed by TLC to monitor the controlled conditions. It may be convenient to protect the 6-oxo group on the base and especially the 2 amino with conventional protecting groups to forestall misacylation.

Intermediates of the formula IId are conveniently prepared by acylation of a carboxy-protected hydroxy alkanoic acid, typically a 2-hydroxy-1-alkanoic acid, with the appropriate activated and N-protected R₂ derivative, such as N-CBZ valyl or isoleucyl in conjunction with a conventional coupling reagent such as DMAP/DCC or with the amino acid halide. The carboxy protecting group is then removed, for instance by acid hydrolysis and the resulting intermediate is activated as described above or the free acid is unsed in conjunction with a coupling reagent to esterify the nucleoside under conventional esterification conditions.

Compounds of the formula Ia are also conveniently prepared by the methodology in the immediately preceding paragraph, namely esterification of a carboxy protected α-hydroxy, ω-carboxy acid, such as glycollic acid, lactic acid, hydroxybutyric acid etc with the appropriate N-protected R₂ derivative, either as the free acid in conjunction with a coupling agent or activated, for instance to the corresponding acid halide. The carboxy protecting group is removed and the resulting intermediate esterified with the nucleoside with the methodology described above.

Compounds comprising a structure of the formula He or IIf are prepared by carboxy protecting the terminal carboxy groups of the respective dicarboxylic acid, such as L-tartaric acid or L-malic acid, with conventional carboxy protecting groups such as benzoyl. The free hydroxy group (s) are then esterified with conventional esterification techniques, such as DMAP & DCC in DMF with the appropriate N-protected R₂ amino acid, such asN-Boc-L-valyl or N-Boc-L-isoleucyl. The benzoyl carboxy protecting groups are removed and the resulting product is esterified to the 5'-hydroxy function of a monohydric nucleoside, using conventional conditions, such as those in the accompanying Examples. Finally, the free carboxy function is esterified with an R₁ group or, more preferbably a conventional pharmaceutically acceptable ester, such as the ethyl ester.

Compounds comprising an optional linker L₂ may also be prepared by a two stage process. In particular a compound of the formula ClC(=O)OC(R₄)(R₄')Cl can be reacted with the 5'-hydroxy of FLG (optionally protected on the base with conventional protecting groups) as is known in the cephalosporin art. The resulting FLG-5'-O-C(=O)OC(R₄)(R₄')chloride is then reacted with an R₁ and R₂ bearing trifunctional linker wherein the third function comprises a carboxyl function, such as the potassium salt.

It will be appreciated that trifunctional L₁ groups of formula IIa wherein and n and m are I and Alk is absent can be prepared from glycerol by regioselective esterification as depicted below in scheme 1 by reference to a stearoyl/L-valyl combination. In short R₁ and R₂ are regioselectively esterified to positions 1 and 3 of the glycerol and position 2 is then converted to the appropriate - T-C(=O)- group, which is then esterified to the 5'-position of the fluoronucleoside or to a cooperating function on L₂ (not depicted). Alternatively the hydroxy at position 2 of the glycerol derivative can be esterified with an L₂ group containing a cooperating carbonyl function on its left hand end.

L₁ groups of formula IIa wherein m is 1, n is 0 and Alk is methylene can also be prepared from glycerol by regioselectively esterifying R₁ and R₂ to positions 1 and 2 of the glycerol, as also depicted below in scheme 1, followed by conversion of the hydroxy at position 3 to the appropriate -T-C(=O)-group. The leftmost series of reactions on Scheme 1 shows the situation where R₁ is esterified to position 1 of the glycerol and R₂ is esterified to position 2. The corresponding arrangement where R₁ is esterified to position 2 and R₂ to position 1 can be achieved by first treating the glycerol with CBz-L-valine/DCC/DMAP/DMF and then protecting the 3 position with pixyl chloride prior to esterifying the fatty acid of R₁ to position 2 of the glycerol, deprotecting and converting the 3 position as necessary.

Although Scheme 1 has been illustrated by reference to a combination wherein R₁ is stearoyl and R₂ is L-valyl, it will be appreciated that this basic scheme will also be applicable to other amino acids, or using conventional protection groups, to combinations of R₂ as an amino acid derivative and R₁ as hydroxy. Linkers where T comprises an -NH- group can be prepared by analogous regioselective esterification followed by conversion of the free hydroxyl to amine, reduction to azide and reaction with phosgene to form the corresponding chlorocarbamate.

A variation of scheme I allowing the preparation of linkers of the formula IIc. In this variation, the phosgene step shown above is replaced by reaction with an activated dicarboxylic acid, such as succinic anhydride. This results in a glycerol triester (comprising the (optionally protected) R₁ ester, the protected R₂ ester and the ester of the dicarboxylic acid) and the free carboxy on the dicarboxylic acid is then activated and esterified to the nucleoside in a conventional fashion. Alternatively linkers of formula IIc can be built up in situ on the nucleoside. In this variant, the dicarboxylic acid is esterified to a suitably protected glycerol derivative. This succinyl monoester is then esterified to the 5'-hydroxy function of the nucleoside in a conventional manner. Finally one or both of the protecting groups on the glycerol moiety is replaced with the L-amino acid ester, and, if present, the remaining protecting group is replaced with a fatty acid 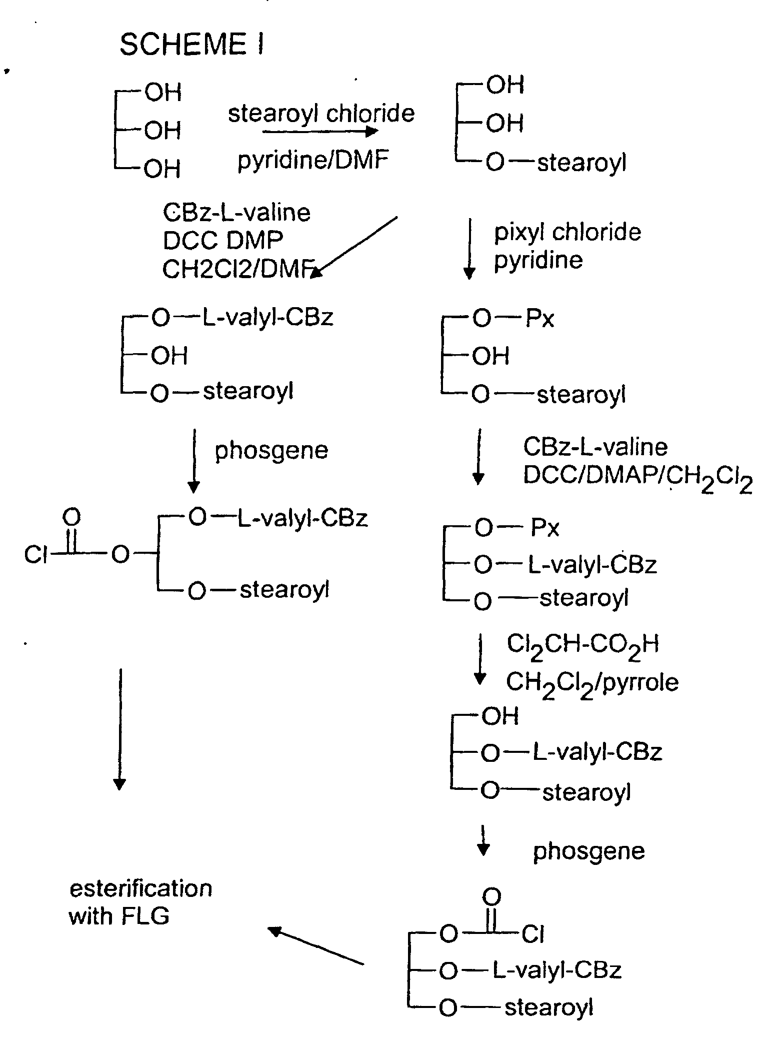 ester or removed to leave a free hydroxy group. This is depicted in Scheme IA which depicts an example wherein the nucleoside in acyclovir (FLG shown in shadow), the dicarboxylic acid is succinyl and R₁ and R₂ are both CBZ-protected valyl, but will, of course be applicable to other variations of Formula Ic. In each case coupling conditions means standard esterification conditions such as coupling reagents DMAP, DCC etc or alternatively conversion of the relevant carboxy function to an activated derivative such as the acid chloride or the activated succinic moiety can also comprise the anhydride.

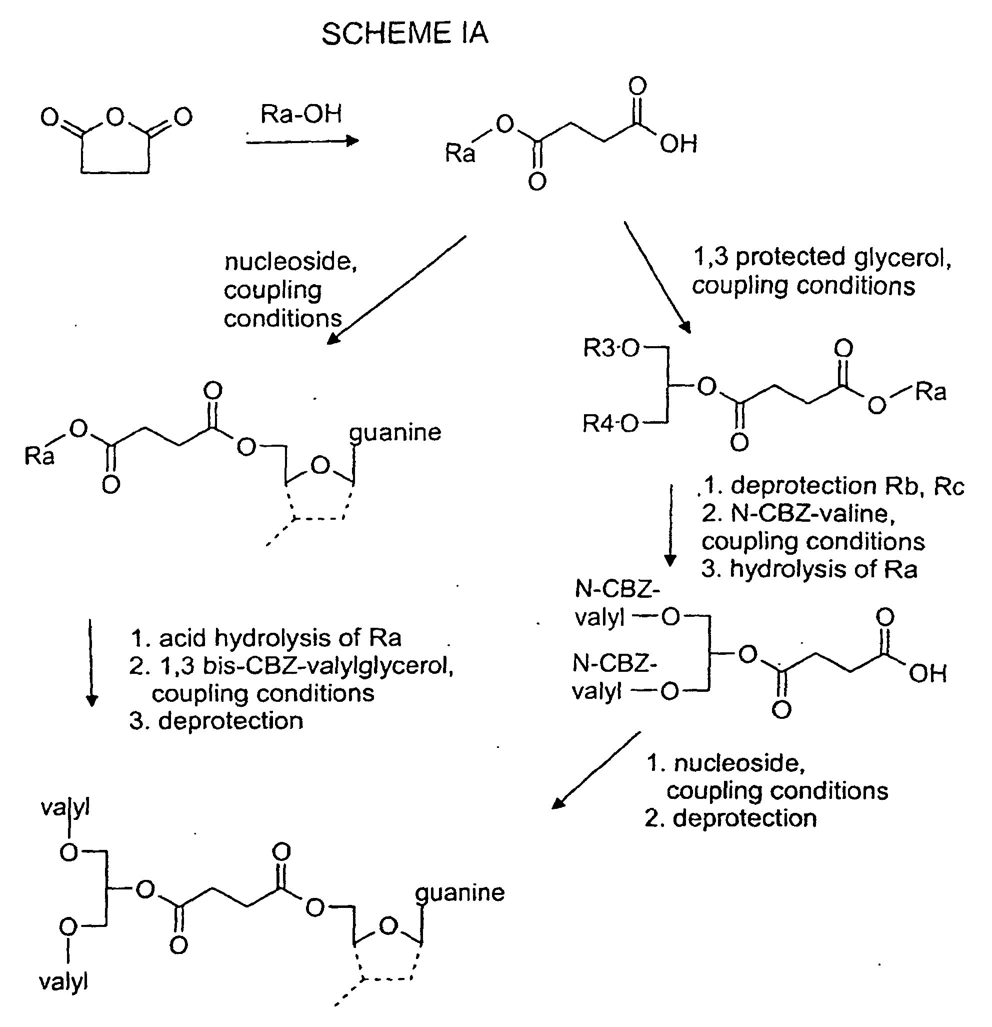

In a variation of Scheme IA, the succinic anhydride is reacted directly with the nucleoside, thus avoiding the first protection and deprotection steps. A further alternative is to regioselectively esterify the glycerol moiety with the N-protected amino acid moiety (ies), generally in conjunction with protection of the hydroxy function intended for coupling to the nucleoside, followed by deprotection of that hydroxy and coupling to the nucleoside.

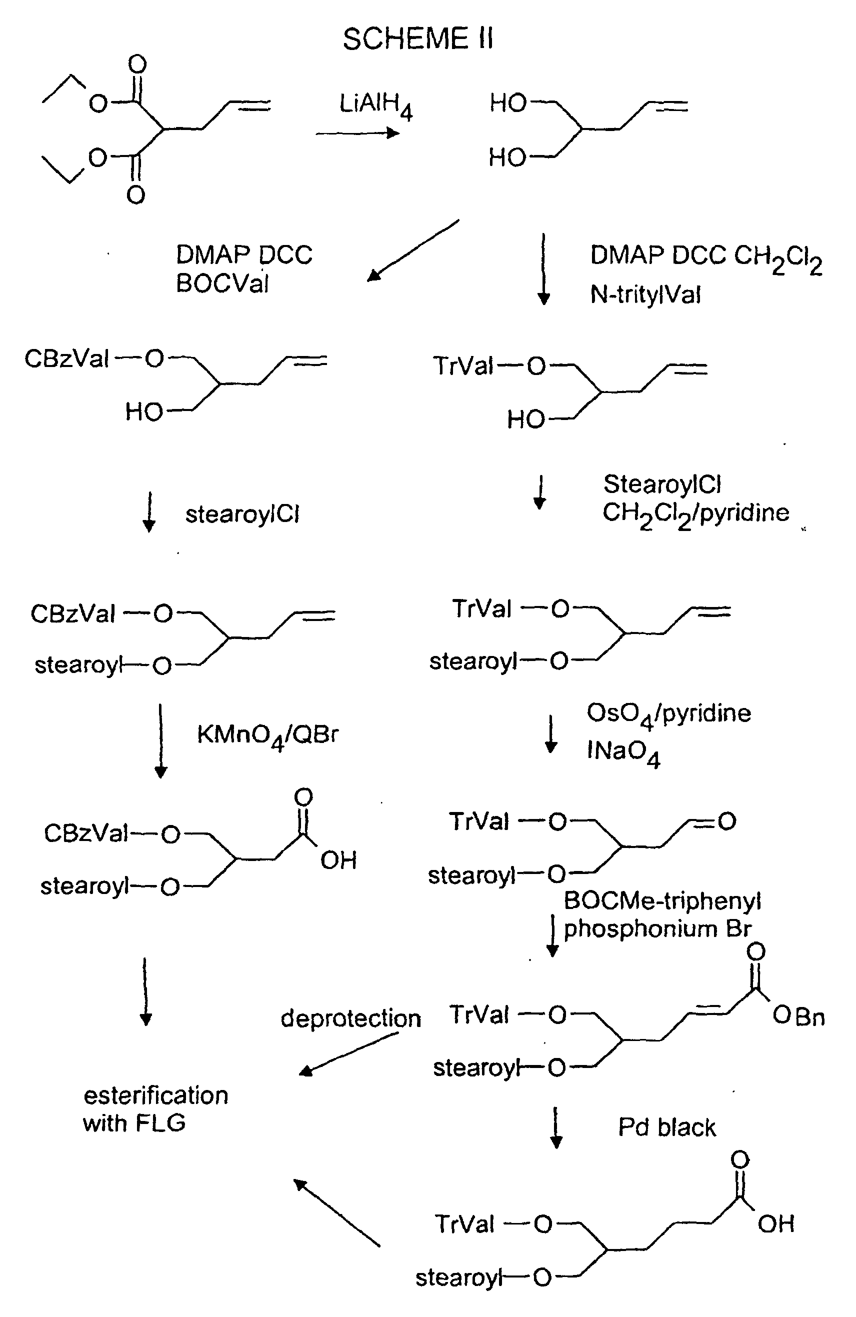

Linkers where m and n are 1, Alk is alkylene or alkenylene and T is a bond can be prepared as shown in Scheme II above. Other permutations of m, n, Alk and the various functions in the trifunctional linker group L₁ of formula IIa can be prepared analogously to the above with the corresponding starter materials, such as 1,2,4-trihydroxybutane (CA registry number 3968-00-6), 3,4-dihydroxybutanoic acid (1518-61-2 & 22329-74-4), (S)-3,4-dihydroxybutanoic acid (51267-44-8), (R)-3,4-dihydroxybutanoic acid (158800-76-1), 1,2,5-pentanetriol (51064-73-4 & 14697-46-2), (S)-1,2,5-pentanetriol (13942-73-9), (R)-1,2,5-pentanetriol (171335-70-9), 4,5-dihydroxypentanoic acid (66679-29-6 & 129725-14-0), 1,3,5-pentanetriol (4328-94-3) and 3-(2-hydroxyethyl)-1,5-pentanediol (53378-75-9). The preparation of each of these starting materials is described in the references to the respective registry number. Ohsawa et al in Chem Pharm Bull 41 (11) 1906-1909 (1993) and Terao et al Chem. Pharm. Bull. 39(3) 823-825 (1991) describe the control of the sterochemistry of trifunctional linker groups with lipase P.

The amino acid derivative of R₂ and, if present, R₁ can alternatively be esterified to the linker group with the 2-oxa-4-aza-cycloalkane-1,3-dione methodology described in international patent application no. WO 94/29311, the contents of which are hereby incorporated by reference.

### Brief Description of the Drawing

Various aspects of the invention will now be described by way of example only with reference to the following Examples and the accompanying drawings in which;
Figure 1 depicts serum viral-DNA levels in treated and untreated, DHBV-infected ducks as a function of time, as described in Biological Example 3;
Figure 2 depicts weight gain in treated, DHBV-infected ducks as a function of time, as described in Biological Example 3.

### EXAMPLE 1

### 2', 3'-Dideoxy-3'-fluoro-5'-O-[3,3-bis(L-valyloxymethyl)-prorpionic acid] guanosine

### a) Synthesis of 4,4-bis (N-CBZ-L-valyloxymethyl)-but-l-ene.

To a solution of 2-allyl-1,3-propandiol (2.32g, 20 mmole), N-CBZ-L-valine (10.06g, 40 mmole) and DMAP (0.488g, 4 mmole) in 120ml dichloromethane was added DCC (9.08g, 44 mmole) in portions and the mixture was stirred overnight at room temperature. The mixture was cooled to 5°C and the urethane was filtered. The filtrate was evaporated and the product was isolated by silica gel column chromatography. Yield : 9.0g
¹H-NMR (CDCl₃) 0.89 (m, 12H) 5.11 (s, 2H) 5.73 (m, 1H)

### b) Synthesis of 3,3-Bis (N-CBZ-L-valyloxymethyl)-propionic acid.

To a cooled solution of 4,4-bis (N-CBZ-L-valyloxymethyl)-but-1-ene (14.6g, 25 mmole) and tetrabutylammonium bromide (1.3g, 4 mmole) in 120ml benzene was added 100ml water. Under strong stirring potassium permanganate (15.8g, 100 mmole) was added in portions and the mixture was stirred for 2 hours between 15°C and 20°C . A sodium bisulfite aqueous solution was added to the slurry until the mixture was discolored. The mixture was acidified with 2N hydrochloric acid and extracted four times with ethyl acetate. The organic phase was washed two times with water, dried with sodium sulfate and evaporated under reduced pressure. The product was isolated by silica gel column chromatography. Yield: 7.5g

¹H-NMR (CDCl₃) 0.89 (m, 12H) 2.05 (m, 2H) 2.46 (m, 2H) 2.62 (m, 1H) 4.20 (m, 6H) 5.11 (s, 4H) 5.30 (m, 2H) 7.35 (m, 10H)

### c) Synthesis of 2',3'-dideoxy-3'-fluoro-5'-O-[3,3-bis (N-CBZ-L-valyloxymethyl)-propionyl]-guanosine.

A solution of 2',3'-dideoxy-3'-fluoroguanosine (1.35g, 5 mmole), 3,3-bis (N-CBZ-L-valyloxymethyl)-propionic acid (3.6g, 6 mmole), DMAP (0.061g, 0.5 mmole) and HOBT (0.81g, 6 mmole) was coevaporated two times with DMF and reduced to about 120ml. DCC (1.24g, 6 mmole) was added and the mixture was stirred overnight at room temperature. The mixture was filtered and the solution was evaporated under reduced pressure. Ethyl acetate (200 ml) was added and the organic phase washed twice with 5% acetic acid, 5% sodium hydrogen carbonate and water. The organic phase was dried with sodium sulfate and evaporated under reduced pressure. The product was isolated by silica gel column chromatography. Yield: 2.7g

¹H-NMR (DMSO d-6) 0.88 (m, 12H) 2.00(m, 2H) 2.50-3.00 (m, 2H) 3.90-4.43 (m, 10H) 5.08 (s, 4H) 5.32-5.59 (m, 1H) 6.17 (m, 1H) 6.50 (s, 2H) 7.28 (m, 10H) 7.72 (m, 2H) 7.90 (s, 1H)

### d) Synthesis of 2', 3'-Dideoxy-3'-fluoro-5'-O- [3,3-bis (L-valyloxymethyl)-propionic acid] guanosine.

A solution of 2', 3'-dideoxy-3'-fluoro-5'-O-[3,3-bis (N-CBZ-L-valyloxymethyl)-propionyl] guanosine (2.6g, 3.1 mmole) in 80ml ethyl acetate, 20ml methanol and 20ml acetic acid was hydrogenated with palladium black (0.3g) for two hours under normal pressure. The catalyst was filtered and washed with ethyl acetate and methanol. The solution was evaporated under reduced pressure and the product was isolated as the bisacetate salt by silica gel column chromatography. Yield: 1.2g

¹H-NMR (DMSO d-6) 0.90 (m, 12H) 1.78 (m, 2H) 2.50-3.00 (m, 2H) 3.09 (m, 2H) 4.02-4.45 (m, 8H) 5.34-5.59 (m, 1H) 6.17 (m, 1H) 6.62 (s, 2H) 7.88 (s, 1H)

### EXAMPLE 2

### 2' 3'-Dideoxy-3'-fluoro-5'-O-3-[1,3-bis-(L-valyloxy)-2-propyloxycarbonyl propanoyl]-guanosine

### a) Synthesis of 1,3-dibenzyloxy-2-propyl succinate monoester.

A solution of 1,3-dibenzyloxypropan-2-ol (6.8g, 25 mmole) and succinic anhydride (7.5g, 75 mmole) and DMAP (12.2g, 100 mmole) was stirred for one hour at 60°C. The mixture was evaporated under reduced pressure, acidified with 2N HCl and extracted two times with ethyl acetate. The combined organic phase was washed three times with water, dried with sodium sulfate and evaporated under reduced pressure.
The product was isolated by silica gel column chromatography. Yield: 7.8g

### b) Synthesis of 2',3'-dideoxy-3'-fluoro-5'-O-[3-(1,3-dibenzyloxy-2-propyloxycarbonyl)-propanoyl] guanosine.

A mixture of 2', 3'-dideoxy-3'-fluoroguanosine (1.61g, 6 mmole), HOBT (0.972g, 7.2 mmole), DMAP (73.3mg, 0.6 mmole) and 1,3-dibenzyloxy-2-propyl succinate monoester (2.68g, 7,2 mmole) was coevaporated two times with DMF and reduced to about 150ml. DCC (1.55g, 7.5 mmole) was added and the mixture was stirred 72 hours at room temperature. The mixture was filtered and the solution was evaporated under reduced pressure. Ethyl acetate (200 ml) was added and the organic phase washed twice with 5% acetic acid, 5% sodium hydrogen carbonate and water. The organic phase was dried with sodium sulfate and evaporated under reduced pressure. The product was isolated by silica gel column chromatography. Yield: 3.3g

### c) Synthesis of 2',3'-dideoxy-3'-fluoro-5'-O-[3-(1,3-dihydoxy-2-propyloxy carbonyl)propanoyl]guanosine.

A solution of 2',3'-dideoxy-3'-fluoro-5'-O-[3-(1,3-dibenzyloxy-2-propyloxy carbonyl)propanoyl]guanosine (3.2g, 5.13 mmole) in 50ml ethyl acetate, 50ml methanol and 10ml acetic acid was hydrogenated with palladium black (0.6g) under 40 psi overnight. The catalyst was filtered and washed with methanol, The solution was evaporated under reduced pressure and the product was isolated by silica gel column chromatography. Yield: 1.64g

### d) Synthesis of 2',3'-dideoxy-3'-fluoro-5'-O- {3-[1,3-Bis ( N-CBZ-L-valyloxy)-2-propyloxycarbonyl]propanoyl}guanosine.

A mixture of 2',3'-dideoxy-3'-f!uoro-5'-O-p-(1,3-dihydroxy-2-propyloxy carbonyl)-propanoyl]guanosine (1.93g, 2.93 mmole), N-CBZ-L-valine (1.76g, 7 mmole), HOBT (0.95g, 7 mmole) and DMAP (85.5mg, 0.7 mmole) was coevaporated two times with DMF and reduced to about 60ml. DCC (1.55g, 7.5 mmole) was added and the mixture was stirred overnight at room temperature. The mixture was warmed for four hours at 60°C and then cooled to about 10°C. The mixture was filtered and the solution was reduced under reduced pressure. Ethyl acetate (150 ml) was added and the organic phase was washed twice with 5% acetic acid, 5% sodium hydrogen carbonate and water. The organic phase was dried with sodium sulfate and evaporated under reduced pressure. The product was isolated by silica gel column chromatography. Yield: 1.6g.

### e) Synthesis of 2', 3'-dideoxy-3'-fluoro-5'-O-{3-[1,3-bis-(L-valyloxy)-2-propyloxycarbonyl]-propanoyl}guanosine.

A solution of 2',3'-dideoxy-3'-fluoro-5'-O-{3-[1,3-bis-(N-CBZ-L-valyloxy)-2-propyloxycarbonyl)propanoyl}guanosine(1.6g, 1.75 mmole) in 80ml ethyl acetate, 20ml methanol and 20 ml acetic acid was hydrogenated with palladium black (0.3g) for two hours at room temperature and normal pressure. The catalyst was filtered and washed with methanol. The solution was evaporated under reduced pressure and the product was isolated as the diacetate salt by silica gel column chromatography. Yield: 1.02g

¹H-NMR (DMSO d-6) 0.84 (m, 12H) 1.85(m, 2H) 2.58 (m, 4H) 2.60-3.10 (m, 2H) 3.11 (m, 2H) 3.61-4.39 (m, 7H) 5.19 (m, 1H) 5.35-5.56 (m, 1H) 6.16 (m, 1H) 6.62 (s, 2H) 7.89 (s,1H)

### EXAMPLE 3

### 2',3'-Dideoxy-3'-fluoro-5'-O-{3-[1-(L-valyloxy)-3-hydroxy-2-propyloxy carbonyl]propanoy}guanosine

### a) Synthesis of 2',3'-dideoxy-3'-fluoro-5'-O-{3-[1-(N-CBZ-L-valyloxy)-3-hydroxy-2-propyloxy carbonyl]-propanoyl} guanosine.

A mixture of 2',3'-dideoxy-3'-fluoro-5'-O-[3-(1,3-dihydroxy-2-propyloxy carbonyl)-propanoyl] guanosine (1.3g, 2.93 mmole), N-CBZ-L-valine (1.00g, 4 mmole), HOBT (0.54g, 4 mmole) and DMAP (48.8mg, 0.4 mmole) was coevaporated two times with DMF and reduced to about 60ml. DCC (0.91g, 4.4mmole) was added and the mixture was stirred for 72 hours at room temperature. The mixture was filtered and the solution evaporated under reduced pressure. Ethyl acetate (150 ml) was added and the organic phase washed twice with 5% acetic acid, 5% sodium hydrogen carbonate and water. The organic phase was dried with sodium sulfate and evaporated under reduced pressure. The product was isolated by silica gel column chromatography. Yield: 0.99g

### b) Synthesis of 2', 3'-dideoxy-3'-fluoro-5'-O-{3-[1-(L-valyloxy)-3-hydroxy-2-propyloxycarbonyl]-propanoyl} guanosine.

A solution of 2',3'-dideoxy-3'-fluoro-5'-O-{3-[1-(N-CBZ-L-valyloxy)-3-hydroxy-2-propyloxycarbonyl)-propanoyl}guanosine (0.82g, 1.21 mmole) in 30ml ethyl acetate, 15ml methanol and 15 ml acetic acid was hydrogenated with palladium black (0.15g) for two hours at room temperature and normal pressure. The catalyst was filtered and washed with methanol. The solution was evaporated under reduced pressure and the product was isolated as the acetate salt by silica gel column chromatography. Yield: 0.5g

¹H.NMR (DMSO d-6) 0.84 (m, 6H) 1.86 (m, 1H) 2.58 (m, 4H) 2.63-3.02 (m, 2H) 3.10-4.38 (m, 9H) 5.34-5.55 (m, 1H) 6.16 (m, 1H) 6.56 (s, 2H) 7.90 (s, 1H)

### EXAMPLE 4

### 2',3'-Dideoxy-3'-fluoro-5'-O-3-[2,3-bis-(L-valyloxy)-1-propyloxycarbonyl]-propanoyl-guanosine.

### a) Synthesis of 4-methoxybenzyl succinate monoester.

To a mixture of succinic anhydride (75g, 750 mmole) and 4-methoxybenyl alcohol (69.1g, 500 mmole) in 1,4-dioxane (300ml) was added pyridine (79.1g, 1000 mmole) and the mixture was stirred for five hours at 80°C . The mixture was evaporated under reduced pressure and 600ml of ethyl acetate and 60 ml of acetic acid were added. The organic phase was washed three times with water, dried with sodium sulfate and evaporated under reduced pressure. The product was recrystallized from toluene. Yield: 104 g.

¹H-NMR (DMSO d-6) 2.48 (m, 4H) 3,72 (s, 3H) 5.00 (s, 2H) 6.90 (d, 2H) 7.28 (d, 2H)

### b) Synthesis of succinic acid 2,3-dihydroxy-propyl ester, 4-methoxybenzyl ester.

To a solution of glycerol (23.0g, 250 mmole),. 4-methoxybenzyl succinate monoester (5.96 g, 25 mmole) and DMAP (0.36g, 3 mmole) in DMF (200ml) was added DCC (6.2g 30 mmole) and the mixture was stirred overnight at room temperature. The mixture was evaporated under reduced pressure and 150ml dichloromethane was added. The mixture was filtered and the solution washed twice with water. The water phase was extracted two times with dichloromethane and the combined organic phases were dried with sodium sulfate. The solution was evaporated under reduced pressure and the product was isolated by silica gel column chromatography.
Yield: 3.0g

¹H-NMR (CDCl₃) 2.65 (m, 4H) 3.61 (m, 2H) 3.80 (s, 3H) 3.90 (m, 1H) 4.18 (m, 2H) 5.05 (s, 2H) 6.89 (d, 2H) 7.26 (d, 2H)

### c) Synthesis of succinic acid 2,3-bis-(N-CBZ-L-Valyloxy)-propyl ester, 4-methoxybenzyl ester.

To a stirred solution of succinic acid 2,3-dihydroxy-propyl ester, 4-methoxybenzyl ester (2.9g, 9.28 mmole), N-CBZ-L-valine (5.03g, 20 mmole) and DMAP (0.244g, 2 mmole) in dichloromethane (60ml) was added DCC (4.5g, 22 mmole) and the mixture was stirred overnight at room temperature. The mixture was filtered and the solution was evaporated under reduced pressure. The product was isolated by silica gel column chromatography. Yield: 2.5g

¹H-NMR (CDCl₃) 0.90 (m, 12H) 2,16 (m, 2H) 2.62 (m, 4H) 3.80 (s, 3H) 4.32 (m, 4H) 5.05-5.52 (m, 9H) 6.89 (d, 2H) 7.30 (m, 12H)

### d) Synthesis of succinic acid 2,3-bis-(N-CBZ-L-valyloxy)propyl ester.

To a solution of the above intermediate (2.3g, 2.95 mmole) in dichloromethane (25ml) was added trifluoroacetic acid (2.5ml) and the solution was stirred for two hours at room temperature. The solution was evaporated under reduced pressure and the product was isolated by silica gel column chromatography. Yield: 1,8g

¹H-NMR (CDCl₃) 0.92 (m, 12H) 2.12 (m, 2H) 2.64 (m, 4H) 4.32 (m, 4H) 5.10 (s, 4H) 5.22-5.50 (m, 3H) 7.34 (m, 10H)

### e) Synthesis of 2',3'-dideoxy-3'-fluoro-5'-O-{3-[2,3-bis(N-CBZ-L-valyloxy)-1-propyloxycarbonyl]propanoyl} guanosine.

A mixture of 2', 3'dideoxy-3'-fluoroguanosine (0.538g, 2 mmole), HOBT (0.327g, 2.42 mmole), DMAP(29.3mg, 0.24 mmole) and succinic acid 2,3-bis-(N-CBZ-L-valyloxy)-1-propyl ester (1.6g, 2.42 mmole) was coevaporated two times with DMF and reduced to about 50ml. DCC (0.536g, 2.6 mmole) was added and the mixture was stirred 72 hours at room temperature. The mixture was filtered and the solution was evaporated under reduced pressure. 100ml of ethyl acetate was added and the organic phase washed twice with 5% acetic acid, 5% sodium hydrogen carbonate and water. The organic phase was dried with sodium sulfate and evaporated under reduced pressure. The product was isolated by silica gel column chromatography. Yield: 0.65g.

¹H-NMR (DMSO-d6) 0.88 (m,12H) 2.08 (m,2H) 2.58-3.04 (m, 6H) 3.92 (m, 2H) 4.10-4.46 (m, 7H) 5.00 (s, 4H) 5.22 (m, 1H) 5.32-5.56 (m, 1H) 6.17 (m, 1H) 6.50 (s, 2H) 7.32 (m, 10H) 7.70 (d, 2H) 7.92 (s,1H)

### f) Synthesis of 2',3'-dideoxy-3'-fluoro-5'-O- {3-[2,3-bis-(L-valyloxy)-1-propyloxycarbonyl]-propanoyl} guanosine.

A solution of the intermediate immediately above (0.57g, 0.626 mmole) in 20ml ethyl acetate, 10ml methanol and 10 ml acetic acid was hydrogenated with palladium black (0.1g) for two hours at room temperature and normal pressure. The catalyst was filtered and washed with methanol. The solution was evaporated under reduced pressure and the product was isolated by silica gel column chromatography. The product was dissolved in dichloromethane and 1M hydrogen chloride in ether (1.1ml) was added. The mixture was evaporated under reduced pressure and dried in vacuo to yield the title compound as the dihydrochloride salt. Yield: 0.37g

¹H-NMR (DMSO d-6) 0.92 (m, 12H) 2.12 (m, 2H) 2.58-3.04 (m, 6H) 3.75 (m, 2H) 4.16-4.50 (m, 7H) 5.19-5.60 (m, 2H) 6.18 (m, 1H) 6.76 (s, 2H) 7.92 (s, 1H)

### EXAMPLE 5

### 2',3'-Dideoxy-3'-fluoro-5'-O-3-[1,3-bis-(L-valyloxy)-2-propyloxycarbonyl]-propanoyl guanosine, dihydrochloride salt.

### a) Synthesis of succinic acid 1,3-dibromo-2-propyl ester, 4-methoxybenzyl ester.

To a solution of 1,3-dibromopropan-2-ol (21.8g, 100 mmole), succinic acid 4-methoxybenzyl ester (28.6g,120 mmole) and DMAP (1.22g, 10 mmole) in dichloromethane (400ml) was added DCC (24.8g, 120 mmole) in portions at about 10°C. The mixture was stirred overnight at room temperature and cooled to about 5°C. The mixture was filtered and the solution was evaporated under reduced pressure. 600ml of ethyl acetate was added and the organic phase was washed twice with 5% acetic acid, 5% sodium hydrogen carbonate and water. The solution was dried with sodium sulfate and evaporated under reduced pressure. The product was isolated by silica gel column chromatography. Yield: 34.8g.

¹H-NMR (CDCl₃) 2.69 (m, 4H) 3.57 (m, 4H) 3.81 (s, 3H) 5.07 (s, 2H) 5.14 (m, 1H) 6,88 (d, 2H) 7.26 (d, 2H)

### b) Synthesis of succinic acid 1,3-bis-(N-CBZ-L-valyloxy)-2-propyl ester, 4-methoxybenzyl ester.

To a solution of N-CBZ-L-valine ( 58.5 g, 232.8 mmole) in dried DMF (300ml) was added potassium-tert.-butoxide (24,68 g, 220 mmole) and the mixture was stirred for one hour at room temperature. A solution of succinic acid 1,3-dibromo-2-propyl ester, 4-methoxybenzyl ester (34 g, 77.6 mmole) in dried DMF (50ml) was added and the mixture was stirred for eighteen hours at 60°C. The potassium bromide was filtered and the solution was evaporated under reduced pressure. 600ml of ethyl acetate was added and the organic phase washed twice with 5% sodium hydrogen carbonate and with water. The organic phase was dried with sodium sulfate and evaporated under reduced pressure. The product was isolated by silica gel column chromatography. Yield: 45g

¹H-NMR (CDCl₃) 0.90 (m, 12H) 2.16 (m, 2H) 2.61 (m, 4H) 3.80 (s, 3H ) 4.12-4.42 (m, 6H) 5.02 (s, 2H) 5.10 (s, 4H) 5.43 (m, 3H) 6.88 (d, 2H) 7.32 (m, 12H)

### c) Synthesis of succinic acid 1,3-bis-(N-CBZ-L-valyloxy)-2-propyl ester.

To a cooled solution of the intermediate immediately above (44.5 g, 57.1 mmole) in dichloromethane (500ml) was added trifluoroacetic acid (50ml) between 5°C and 10°C and the solution was stirred for two hours at 10°C. The solution was evaporated under reduced pressure and two times coevaporated with toluene. 400ml of ethanol was added and the mixture was stirred for 30 minutes at 40°C. The mixture was cooled and the by-product filtered. The solution was evaporated under reduced pressure and the product was isolated by silica gel column chromatography. Yield: 33g

¹H-NMR (DMSO-d6) 0.88 (m, 12H) 2.04 (m, 2H) 2.46 (m, 4H) 3.94-4.40 (m, 6H) 5.02 (s, 4H) 5.18 (m, 1H) 7.32 (m, 10H) 7,74 (d , 2H)

### d) Synthesis of 2',3'-dideoxy-3'-nuoro-5'-0-{3-[1,3-bis-(N-CBZ-L-valyloxy)-2-propyloxycarbonyl]propanoyl} guanosine.

A mixture of 2',3'dideoxy-3'-fluoroguanosine (17.8 g, 66 mmole), HOBT (10.64 g, 78.8 mmole), succinic acid 1,3-*bis*-(N-CBZ-L-valyloxy)-2-propyl ester (52 g, 78.8 mmole) and DMAP (0.96 g, 7.88 mmole) was coevaporated two times with DMF and reduced to about 500ml. DCC (17.3 g, 84 mmole) was added and the mixture was stirred overnight at room temperature. The mixture was warmed for six hours at 60°C and then cooled to about 10°C. The mixture was filtered and the solution was reduced under reduced pressure. 1200 ml of ethyl acetate was added and the organic phase was washed twice with 5% acetic acid, 5% sodium hydrogen carbonate and water. The organic phase was dried with sodium sulfate and evaporated under reduced pressure. The product was isolated by silica gel column chromatography. Yield: 42g.

¹H-NMR (DMSO-d6) 0.90 (m, 12H) 2.02 (m, 2H) 2.5-3.02 (m, 6H) 3.94 (m, 2H) 4.22 (m, 7H) 5.02 (s, 4H) 5.18 (m, 1H) 5.22-5.50 (m, 1H) 6.16 (m, 1H) 6.50 (s, 2H) 7.32 (m, 10H) 7.72 (d, 2H) 7.92 (s, 1H)

### e) Synthesis of 2',3'-dideoxy-3'-fluoro-5'-O-{3-[1,3-bis-(L-valyloxy)-2-propyloxycarbonyl]-propanoyl}guanosine dihydrochloride salt.

A solution of 2',3'-dideoxy-3'-fluoro-5'-O-{3-[1,3-*bis*-(N-CBZ-L-valyloxy)-2-propyloxy carbonyl]propanoyl} guanosine (5.0, 5.9 mmole) in 75ml ethyl acetate and 75ml methanol was hydrogenated with palladium on activated carbon 10% Pd (1g) one hour at room temperature and normal pressure. The catalyst was filtered and washed with methanol. The solution was evaporated under reduced pressure. The product was dissolved in dichloromethane and a solution of 1M hydrogen chloride in ether (6ml) was added, while cooling. The mixture was evaporated under reduced pressure. Yield: 3.5g

¹H-NMR (DMSO d-6) 0.94 (m, 12H) 2.18 (m, 2H) 2.5-3.04 (m, 6H) 4.20-4.54 (m, 7H) 5.24 (m, 1H) 5.34-5.64 (m, 1H) 6.22 (m, 1H) 6.92 (s,2H) 8.30 (s, 1H) 8.62 (s, 6H)

### EXAMPLE 6

### Alternative synthesis of 2',3'-dideoxy-3'-fluoro-5'-O- 3-[1,3-bis-(L-valyloxy)-2-propyloxycarbonyl] propanoyl guanosine

### a) Synthesis of succinic acid 1,3-dibromo-2-propyl ester, 1,1-dimethylethyl ester.

To a solution of 1,3-dibromopropan-2-ol (10.9 g 50 mmole), succinic acid 1,1-dimethylethyl ester (J. Org. Chem. 59 (1994) 4864) (10.45g, 60 mmole) and DMAP (0.61 g, 5 mmole) in dichloromethane (180ml) was added DCC (12.4 g, 60 mmole) in portions at about 10°C. The mixture was stirred overnight at room temperature and cooled to about 5°C. The mixture was filtered and the solution was evaporated under reduced pressure. 250ml ethyl acetate was added and the organic phase was washed twice with 5% citric acid, 5% sodium hydrogen carbonate and water. The solution was dried with sodium sulfate and evaporated under reduced pressure. The product was distilled in vacuo. (bp 0,5 135-140°C) Yield: 16.8 g

¹H-NMR (CDCl₃) 1.45 (s, 9H) 2.58 (m, 4H) 3.61 (m, 4H) 5.12 (m, 1H)

### b) Synthesis of succinic acid 1,3-bis-(N-CBZ-L-valyloxy)-2-propyl ester, 1,1-dimethylethyl ester.

To a solution of N-CBZ-L-valine (18.85 g, 75 mmole) in dried DMF (100ml) was added potassium tert.-butoxide (7.85 g, 70 mmole) and the mixture was stirred for one hour at room temperature. A solution of succinic acid 1,3-dibromo-2-propyl ester, 1,1-dimethylethyl ester (9.35g, 25 mmole) in dried DMF (20ml) was added and the mixture was stirred for eighteen hours at 60°C. The potassium bromide was filtered and the solution evaporated under reduced pressure. 300ml of ethyl acetate were added and the organic phase washed twice with 5% sodium hydrogen carbonate and with water. The organic phase was dried with sodium sulfate and evaporated under reduced pressure. The product was isolated by silica gel column chromatography. Yield: 14g

¹H-NMR (CDCl₃) 0.90 (m, 12H) 1.42 (s, 9H) 2.14 (m, 2H) 2.52 (m, 4H) 4.32 (m, 6H) 5.10 (s, 4H) 5.32 (m, 3H) 7.26 (m, 10H)

### c) Synthesis of 1,3-bis-(N-CBZ-L-valyloxy )-2-propyl succinate monoester.

To a cooled solution of succinic acid 1,3-*bis*-(N-CBZ-L-valyloxy)-2-propyl ester, 1,1-dimethylethyl ester (13 g, 18.18 mmole) in dichloromethane (100ml) was added trifluoroacetic acid (20ml) and the solution was stirred for six hours at room temperature. The solution was evaporated under reduced pressure. 200ml ethyl acetate was added and the organic phase was washed with 5% sodium hydrogen carbonate and water. The solution was evaporated under reduced pressure.
Yield: 11.7g

¹H-NMR (DMSO-d6) 0.88 (m, 12H) 2.04 (m, 2H) 2.46 (m, 4H) 3.94-4.40 (m, 6H) 5.02 (s, 4H) 5.18 (m, 1H) 7.32 (m, 10H) 7.74 (d, 2H)

### d) Synthesis of 2',3'-dideoxy-3'-fluoro-5'-O- 3-[1,3-bis-(L-valyloxy)-2-propyloxycarbonyl] propanoyl guanosine

The intermediate from step c) is esterified to FLG as shown in example 5 step d) of EP-A 1 123 935 and the N-protecting groups on the valyl moieties removed by conventional techniques, such as shown in Example 6 step e) or Example 2 step e).

### BIOLOGICAL EXAMPLE 1

### Pharmacokinetics

Confirmation that orally administered prodrugs of the invention release FLG in vivo is obtained in a rat model which is recognised as a useful model for assessing pharmacokinetic parameters of nucleoside analogues. The oral compositions are administered in a pharmaceutical vehicle comprising propylene glycol, or in the case of the more soluble compounds such as that of Example 1 or Example 5, in water, to duplicate fasted animals in a dosage corresponding to 0.1 mmole/kg. For comparison, a set of rats is iv dosed with 0.01 mmole/kg of the metabolite 2',3'-dideoxy-3'-fluoroguanosine. Serum levels of the metabolite are then monitored in serum collected at intervals from individual animals from 0.5 to up to 12 hours following administration (5 min to 6 hours for FLG).

The metabolite is analysed with HPLC with UV detection at 254 nm, in a manner analogous to Ståble et al 1995, J Pharm. Biomed. Anal. 13, 369-376. An HPLC system can be based on a 0.05 M ammonium-dihydrogen-phosphate buffer, with 1.2 % 2-propanol solvent, buffered to pH 4.5 or 30 mM sodium dihydrogen phosphate buffer with 2% acetonitrile solvent buffered to pH 7.0. The column may be a 100 x 2.1 mm BAS C18 5 µm particle size with a 7 µm C18 guard column or Zorbax SB-CN C18 150x4.6mm, 5µm column. Protein binding of the compounds of

**Table 1**

| | 6h absolute bioavail. % | 12h absolute bioavail. % |
|---|---|---|
| FLG | | 9%** |
| Example 1 | | 67.5% |
| Example 2 | 51% | |

| | | |
|---|---|---|
| **literature value | | |

the invention is negligible as is that of the metabolite and ultrafiltration through Amicon or Microcon 30 filters is useful for serum samples. Advantageously the main peak is subject to further column chromatography to better aid in resolution of FLG over low weight serum components. The iv levels are multiplied by a factor of ten in order to obtain AUC values for comparison with the oral values. Absolute oral bioavailability is determined as the ratio between ^{0-∞}AUCᵢᵥ and ^{0-∞}AUCₒᵣₐₗ.

The compounds of the invention thus provide significantly enhanced oral bioavailability relative to the metabolite 2',3'-dideoxy-3'-fluoroguanosine. Notably, the compounds are released into the blood in a relatively sustained manner, rather than in an immediate peak. This means that effective amounts of the active metabolite are available in the blood for many hours assisting once daily dosage. Additionally, a sustained release avoids the problems of acute toxicity seen in compounds with a more rapid release rate.

Although the rat is well recognised as a good model for predicting human bioavailability of nucleoside analogues, species independent bioavailability of a compound of the invention (Example 5) was confirmed in ≅ 11.5 kg male and female beagle dogs administered orally with 0.05 mmole/kg (38 mg/kg) compound in water or iv 0.005 mmole/kg (1.35 mg/kg) metabolite in water. Plasma collection and analysis as above.

| | | |
|---|---|---|
| Male dog | 12 hour absolute bioavailability | 51% |
| Female dog | 12 hour absolute bioavailability | 74% |

### Antiviral activity - Retroviruses

As can be demonstrated by the methodology of Biological Example 1, the compounds of the invention release, *in vivo,* the metabolite 2',3'-dideoxy, 3'-fluoroguanosine. *In vitro* measurement of the antiviral activity of this metabolite will thus reflect the *de facto* activity of the compounds of the invention.

In the XTT dye uptake assay ofKoshida et al. Antimicrob Agents Chemother. 33 778-780, 1989) utilising MT4 cells, the metabolite measured in Biological Example 1 above showed the following *in vitro* activities against retroviruses:

**Table 2**

| HIV or retroviral strain | IC₅₀* |
|---|---|
| HIV-l_{111B} | 1 µg/ml |
| HIV-1²⁴⁴¹ AZT^{r} | 1 µg/ml |
| HIV-1_{111B}TIBO^{r} | 1 µg/ml |
| HIV-1^{29/9} | 0.7 µg/ml |
| HN-2_{SBL6669} | 2 µg/ml |
| SIV_{SM} | 1 µg/ml |

| | |
|---|---|
| *Concentration of metabolite inducing 50% inhibition of viral replication | |

It will thus be apparent that administration of the compounds of the invention induce powerful antiviral activities against the retroviruses HIV-1, HIV-2 and SIV. It should also be noted from the HIV-1²⁴⁴¹ AZT^{r} and HIV-1_{111B} TIBO^{r} results that the antiviral activity of the compounds of the invention does not show cross resistance against strains ofHIV which have become resistant to other HIV agents such as the nucleoside analogue AZT or or the non-nucleoside reverse transcriptase inhibitor TIBO.

### BIOLOGICAL EXAMPLE 3

### Antiviral activity-HBV

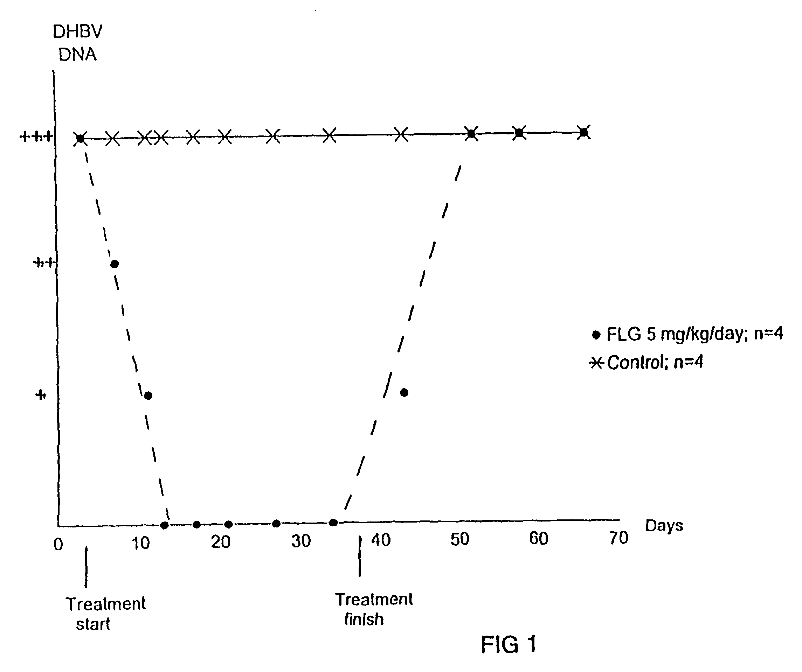

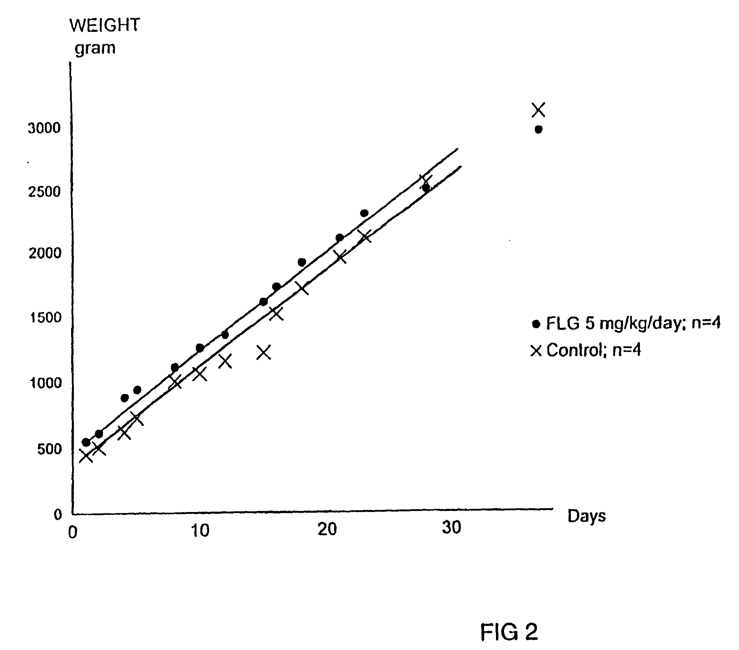

The activity of antivirals on duck hepatitis B virus (DHBV) in ducks is an acknowledged animal model for the validation of *in vivo* hepatitis B activity in humans. The activity of the *in vivo* metabolite measured in Biological Example 2 above has been assayed in the DHBV model described by Sherker et al (1986) Gastroenterology 91, pp 818-824. The results are depicted in Figures 1 and 2 In short, 4 control ducks were treated with phosphate buffered saline (PBS) and 4 ducks with 5 mg/kg/day of the active metabolite. The ducks were two days old when inoculated with DHBV and 18 days old when treatment was commenced. The metabolite and PBS (controls) were given intraperitoneally for 10 days as twice daily injections, at 8 am and 4 pm. Treatment lasted 33 days and the animals were followed 5 weeks after the end of treatment.

The efficacy of treatment was followed by dot blot-hybridisation of DHBV DNA in serum using a radioactive probe and the amount of DHBV measured as the amount of radioactivity hybridised. Figure 1 plots the amount of DHBV DNA in serum at different timepoints before, during and after treatment.

As can be seen in Figure 1, there is no decrease in the amount of DHBV in serum during treatment with PBS (control, solid line). The animals given the metabolite measured in Biological Example 2 (broken line) showed a dramatic decrease in the amount of DHBV in serum during the first 10 days of treatment, whereupon for the remainder of treatment the level of DHBV DNA was below the detection limit at this dose of 5 mg/kg/day. Repeat experiments at dosages of 30 and 3 mg/kg/day and with congenitally infected ducks (not shown) also produced similar results, that is a dramatic fall in serum DHBV DNA to under the detection threshold. Even at the very low dose of 0.3 mg/kg/day the metabolite caused a considerable inhibition of DHBV *in vivo.* After the finish of treatment, virus reappeared in the serum, as shown in Fig. 1. Reappearance ofHBV after short term treatment with conventional antivirals has been observed earlier in both humans and animals with chronic hepatitis B infection.

As can be seen in Fig 2, the weight of the ducks increased in the same way as in the control (PBS treated) animals. The weight increase from about 270 g to about 800 g which was observed during the treatment period is so large that toxic effects, had they occurred, should be easily visible as a change in growth rate. Similar growth curves were also observed for the ducks receiving the higher dosage rate of 30 mg/kg/day. This metabolite is thus clearly non-toxic. As the compounds of the invention are hydrolysed *in vivo* to give this metabolite, and a nature identical and therefore easily metabolised fatty acid, it can therefore be inferred that no long term toxicity problem can be expected from administration of the compounds of the invention. The absence of acute (short term) toxicity of the compounds of the invention when administered orally is established in Biological Example 2 above.

## Claims

1. A compound of formula I 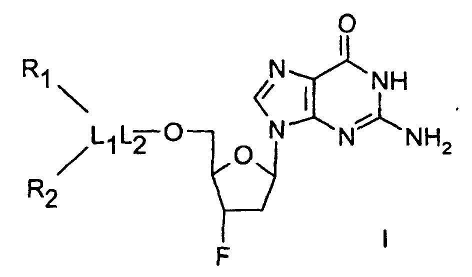 wherein:
R₁ is hydroxy; optionally having esterified thereon an aliphatic L-amino acid;
R₂ is the residue of an aliphatic L-amino acid esterified to an hydroxy function on L1;
L₁ is a trifunctional linker group, wherein one function is R₁ and a second function is an hydroxy group to which R₂ is esterified;
L₂ is absent or a dicarboxylic acid derivative;
wherein the third function on L1; or, if present, a carboxy function on L2; is a carboxy group esterified to the adjacent 5'-O function;
and pharmaceutically acceptable salts thereof.

2. A compound according to claim 1, wherein R₂ is derived from L-alanine, L-leucine, L-isoleucine and preferably L-valine.

3. A compound according to claim 2, wherein both R₁ and R₂ comprise the same L-amino acid.

4. A compound according to any of the preceding claims, wherein L1 is a glycerol derivative.

5. A compound according to claim 4, wherein the dicarboxylic acid derivative comprises oxalyl, malonyl, succinyl, glutaryl or adipyl, preferably succinyl.

6. A compound according to claim 5, wherein R₁ and R₂ are both L-valyl or L-isoleucyl.

7. A compound according to claim 1, denoted 2', 3'-dideoxy-3'-fluoro-5'-O-3-[2,3-bis-(L-valyloxy)-1-propyloxycarbonyl]-propanoyl guanosine or 2', 3'-dideoxy-3'-fluoro-5'-O-3-[1,3-bis-(L-valyloxy)-2-propyloxycarbonyl]-propanoyl guanosine or a pharmaceutically acceptable salt thereof.

8. A compound according to claim 7 denoted 2', 3'-dideoxy-3'-fluoro-5'-O-3-[1,3-bis-(L-valyloxy)-2-propyloxycarbonyl]-propanoyl guanosine hydrochloride.

9. A pharmaceutical composition comprising a compound as defined in any one of claims 1 to 8 and a pharmaceutically acceptable carrier or diluent therefor.

10. The pharmaceutical composition according to claim 9, wherein the compound is 2', 3'-dideoxy-3'-fluoro-5 '-O-3-[2,3-bis-(L-valyloxy)-1-propyloxycarbonyl]-propanoyl guanosine, 2', 3'-dideoxy-3'-fluoro-5'-O-3-[1,3-bis-(L-valyloxy)-2-propyloxycarbonyl]-propanoyl guanosine, or a pharmaceutically acceptable salt thereof

11. A compound or salt as defined in any one of claims 1 to 8 for use in therapy, preferably in the manufacture of a medicament for the treatment or prophylaxis of HBV or retroviral infections.

12. The compound or salt according to claim 11, wherein the retroviral infection is an HIV. infection.

13. The compound or salt according to claim 11 or 12, wherein the compound is 2', 3'-dideoxy-3'-fluoro-5'-O-3-[2,3-bis-(L-valyloxy)-1-propyloxycarbonyl)-propanoyl guanosine, 2', 3'-dideoxy-3'-fluoro-5'-O-3-[1,3-bis-(L-valyloxy)-2-propyloxycarbonyl]-propanoyl guanosine, or a pharmaceutically acceptable salt thereof.

## Patentansprüche

1. Verbindung der Formel I: 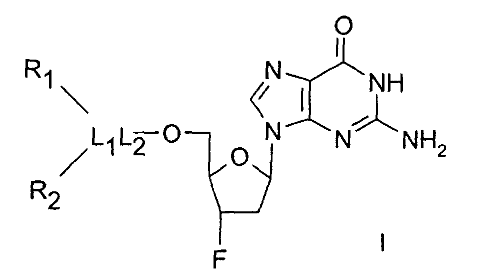 wobei:
R₁ Hydroxy ist; gegebenenfalls mit einer daran veresterten aliphatischen L-Aminosäure;
R₂ ein aliphatischer L-Aminosäure-Rest ist, der mit einer funktionellen Hydroxygruppe von L₁ verestert ist;
L₁ eine trifunktionale Linkergruppe ist, wobei eine funktionelle Gruppe R₁ ist und eine zweite funktionelle Gruppe eine Hydroxygruppe ist, mit der R₂ verestert ist;
L₂ fehlt oder ein Dicarbonsäure-Derivat ist;
wobei die dritte funktionelle Gruppe in L₁; oder, wenn vorhanden, eine funktionelle Carboxygruppe in L₂; eine Carboxygruppe ist, die mit der angrenzenden funktionellen 5'-O-Gruppe verestert ist;
und pharmazeutisch verträgliche Salze davon.

2. Verbindung gemäß Anspruch 1, wobei R₂ von L-Alanin, L-Leucin, L-Isoleucin und vorzugsweise von L-Valin abstammt.

3. Verbindung gemäß Anspruch 2, wobei R₁ and R₂ beide die selbe L-Aminosäure umfassen.

4. Verbindung gemäß einem der vorhergehenden Ansprüche, wobei L1 ein Glyzerin-Derivat ist.

5. Verbindung gemäß Anspruch 4, wobei das Dicarbonsäure-Derivat Oxalyl, Malonyl, Succinyl, Glutaryl oder Adipyl, vorzugsweise Succinyl umfasst.

6. Verbindung gemäß Anspruch 5, wobei R₁ and R₂ beide L-Valyl oder L-Isoleucyl sind.

7. Verbindung gemäß Anspruch 1, bezeichnet als 2',3'-Didesoxy-3'-fluor-5'-O-3-[2,3-bis-(Lvalyloxy)-1-propyloxycarbonyl]-propanoyl-Guanosin oder 2',3'-Didesoxy-3'-fluor-5'-O-3-[1,3-bis-(L-valyloxy)-2-propyloxycarbonyl]-propanoyl-Guanosin oder ein pharmazeutisch verträgliches Salz davon.

8. Verbindung gemäß Anspruch 7 bezeichnet als 2',3'-Didesoxy-3'-fluor-5'-O-3-[1,3-bis-(Lvalyloxy)-2-propyloxycarbonyl]-propanoyl-Guanosinhydrochlorid.

9. Pharmazeutische Zusammensetzung umfassend eine Verbindung wie sie in einem der Ansprüche 1 bis 8 definiert ist und einen pharmazeutisch verträglichen Träger oder ein Verdünnungsmittel dafür.

10. Pharmazeutische Zusammensetzung gemäß Anspruch 9, wobei die Verbindung 2',3'-Didesoxy-3'-fluor-5'-O-3-[2,3-bis-(L-valyloxy)-1-propyloxycarbonyl]-propanoyl-Guanosin, 2', 3'-Didesoxy-3'-fluor-5'-O-3-[1,3-bis-(L-valyloxy)-2-propyloxycarbonyl]-propanoyl-Guanosin oder ein pharmazeutisch verträgliches Salz davon ist.

11. Verbindung oder Salz wie sie/es in einem der Ansprüche 1 bis 8 definiert ist für die Verwendung in der Therapie, vorzugsweise für die Herstellung eines Medikaments für die Behandlung oder Prophylaxe von HBV- oder retroviralen Infektionen.

12. Verbindung oder Salz gemäß Anspruch 11, wobei die retrovirale Infektion eine HIV-Infektion ist.

13. Verbindung oder Salz gemäß Anspruch 11 oder 12, wobei die Verbindung 2',3'-Didesoxy-3'-fluor-5'-O-3-[2,3-bis-(L-valyloxy)-1-propyloxycarbonyl]-propanoyl-Guanosin, 2',3'-Didesoxy-3'-fluor-5 '-O-3-[1,3-bis-(L-valyloxy)-2-propyloxycarbonyl]-propanoyl-Guanosin, oder ein pharmazeutisch verträgliches Salz davon ist.

## Revendications

1. Composé de formule 1 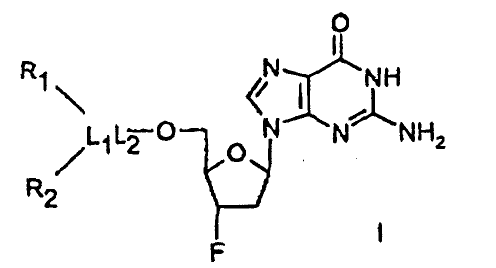 dans lequel :
R₁ est groupe hydroxy ; présentant éventuellement un acide aminé L aliphatique sous une forme estérifiée ;
R₂ est le résidu d'un acide aminé L aliphatique estérifié sur une fonction hydroxy sur L₁;
L₁ est un groupe de liaison trifonctionnel, dans lequel une fonction est R₁ et une seconde fonction est un groupe hydroxy auquel R₂ est estérifié;
L₂ est absent ou est un dérivé d'acide dicarboxylique ;
dans lequel la troisième fonction sur L₁ ; ou, si elle est présente, une fonction carboxy sur L₂ ; est un groupe carboxy estérifé à la fonction 5'-O adjacente;
et leurs sels acceptables sur le plan pharmaceutique.

2. Composé selon la revendication 1, dans lequel R₂ est dérivé de L-alanine, L-leucine, L-isoleucine et de préférence de L-valine.

3. Composé selon la revendication 2, dans lequel R₁ et R₂ comprennent tous deux le même acide aminé L.

4. Composé selon l'une quelconque des revendications précédentes, dans lequel L1 est un dérivé du glycérol.

5. Composé selon la revendication 4, dans lequel le dérivé d'acide dicarboxylique comprend un oxalyle, malonyle, succinyle, glutaryle ou adipyle, de préférence un succinyle.

6. Composé selon la revendication 5, dans lequel R₁ et R₂ sont tous les deux L-valyle ou L-isoleucyle.

7. Composé selon la revendication 1, désigné 2', 3'-didéoxy-3'-fluoro-5'-O-3-[2,3-bis-(L-valyloxy)-1-propyloxycarbonyle]-propanoyle guanosine ou 2', 3'-didéoxy-3'-fluoro-5'-O-3-[1,3-bis-(L-valyloxy)-2-propyloxycarbonyle]-propanoyle guanosine ou un sel acceptable sur le plan pharmaceutique de ceux-ci.

8. Composé selon la revendication 7, désigné hydrochlomre de 2', 3'-didéoxy-3'-fluoro-5'-O-3-[1,3-bis-(L-valyloxy)-2-propyloxycarbonyle]-propanoyle guanosine.

9. Composition pharmaceutique comprenant un composé tel que défini dans l'une quelconque des revendications 1 à 8 et un support ou un diluant pour celui-ci, acceptable sur le plan pharmaceutique.

10. Composition pharmaceutique selon la revendication 9, dans laquelle le composé est le 2', 3'-didéoxy-3'-fluoro-5'-O-3-[2,3-bis-(L-valyloxy)-1-propyloxycarbonyle]-propanoyle guanosine, le 2', 3'-didéoxy-3'-fluoro-5'-O-3-[1,3-bis-(L-valyloxy)-2-propyloxycarbonyle]-propanoyle guanosine ou un sel acceptable sur le plan pharmaceutique de ceux-ci.

11. Composé ou sel tel que défini dans l'une quelconque des revendications 1 à 8 pour une utilisation en thérapie, de préférence dans la préparation d'un médicament pour le traitement ou la prophylaxie d'infections à VHB ou rétrovirale.

12. Composé ou sel selon la revendication 11, dans lequel l'infection rétrovirale est une infection à VIH.

13. Composé ou sel selon la revendication 11 ou 12, dans lequel le composé est le 2', 3'-didéoxy-3'-fluoro-5'-O-3-[2,3-bis-(L-valyloxy)-1-propyloxycarbonyle]-propanoyle guanosine, le 2', 3'-didéoxy-3'-fluoro-5'-O-3-[1,3-bis-(L-valyloxy)-2-propyloxycarbonyle]-propanoyle guanosine ou un sel acceptable sur le plan pharmaceutique de ceux-ci.
